(19) Europäisches Patentamt
European Patent Office
Office européen des brevets

(11) Publication number: 0 097 953
A2

(12) # EUROPEAN PATENT APPLICATION

(21) Application number: 83106282.3

(22) Date of filing: 28.06.83

(51) Int. Cl.$^3$: A 61 K 31/485
//(A61K31/485, 31/19),
(A61K31/485, 31/40), (A61K31/485,
31/405), (A61K31/485, 31/415)

(30) Priority: 30.06.82 US 393807
30.06.82 US 393808
30.06.82 US 393805
30.06.82 US 393803
30.06.82 US 393804
30.06.82 US 393809

(43) Date of publication of application:
11.01.84 Bulletin 84/2

(84) Designated Contracting States:
AT BE CH DE FR GB IT LI LU NL SE

(71) Applicant: E.I. DU PONT DE NEMOURS AND COMPANY
Legal Department 1007 Market Street
Wilmington Delaware 19898(US)

(72) Inventor: Schmidt, William Koch
2519 Duncan Road
Wilmington Delaware 19808(US)

(74) Representative: von Kreisler, Alek, Dipl.-Chem. et al,
Deichmannhaus am Hauptbahnhof
D-5000 Köln 1(DE)

(54) **Synergistic compositions of nalbuphine and non-narcotic analgesic compounds.**

(57) Pharmaceutical compositions of nalbuphine and non-narcotic analgesic compounds such as ibuprofen, zomepirac, indomethacin, tiflamizole, sulindac, and flurbiprofen have been found to exhibit unexpectedly enhanced analgesic activity by applying an analysis model which considers data characterizing the analgesic effect of both the pure components as well as the fixed dose ratio combinations. This synergism enables the use of lower doses of either or both drugs with a concomitant reduction in risk of possible side effects.

EP 0 097 953 A2

Title BP-6213 Cognate

# SYNERGISTIC COMPOSITIONS OF NALBUPHINE AND NON-NARCOTIC ANALGESIC COMPOUNDS

## Technical Field

This invention relates to pharmaceutical compositions of nalbuphine and non-narcotic analgesic compounds such as ibuprofen, zomepirac, indomethacin, tiflamizole, sulindac, and flurbiprofen having analgesic activity in mammals, and to a method of use of such compositions to alleviate pain in mammals.

## Background of the Invention

More active analgesic combinations are in constant demand because they offer the attractive possibility of relieving pain with reduced dosages thereby diminishing the expected side effects and toxicity that would result from the otherwise required higher dosages.

U.S. Patent 3,393,197, issued to Pachter and Matossian on July 16, 1968, discloses N-substituted-14-hydroxydihydronormorphines, including the N-cyclobutylmethyl derivative, commonly called nalbuphine:

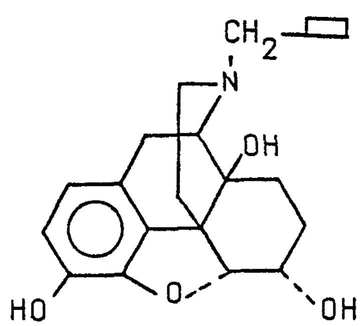

Pachter and Matossian and others, such as H. W. Elliott, et al., J. Med. (Basel), 1, 74-89 (1970); H. Blumberg, et al., Pharmacologist, 10, 189 (Fall 1968); P. Roberts, Drugs of the Future, 2, 613-5 (1977), disclose the use of nalbuphine as an analgesic for the control of moderate to severe pain.

U.S. Patent 4,237,140, issued to J. R. Dudzinski on December 2, 1980, describes an analgesic mixture of nalbuphine and acetaminophen. U.S. Patent 4,282,215, issued to J. R. Dudzinski and W. K. Schmidt on August 4, 1981, describes an analgesic mixture of nalbuphine and aspirin.

U.S. Patents 3,228,831 and 3,385,886 issued to Nicholson and Adams disclose the synthesis, formulation, and analgesic properties of α-methyl-4-(2-methylpropyl)benzeneacetic acid, commonly called ibuprofen:

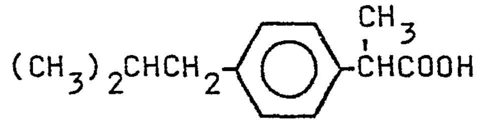

Adams et al., Arch. Pharmacodyn. Ther., 178, 115 (1969), further characterize the use of ibuprofen as an analgesic.

U.S. Patent 3,752,826 issued to J. R. Carson discloses the synthesis and utility of 5-(4-chlorobenzoyl)-1,4-dimethyl-1H-pyrrole-2-acetic acid, commonly called zomepirac:

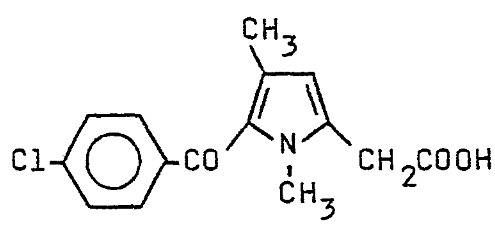

Additional information characterizing the analgesic properties of zomepirac are listed in New Drug Commentary, 8(5), May 1981.

Analgesic mixtures of zomepirac with acetaminophen are disclosed in U.S. Patent 4,260,629, analgesic mixtures of zomepirac with butorphanol are disclosed in U.S. Patent 4,322,427, and mixtures of zomepirac

with aspirin or acetaminophen are disclosed in U.S. Patent 4,132,788.

U.S. Patent 4,190,666, issued to Cherkofsky and Sharpe on February 26, 1980, discloses the synthesis and analgesic properties of a class of compounds including 4,5-bis(4-fluorophenyl)-2-[(1,1,2,2-tetrafluoroethyl)sulfonyl]-1H-imidazole, commonly called tiflamizole:

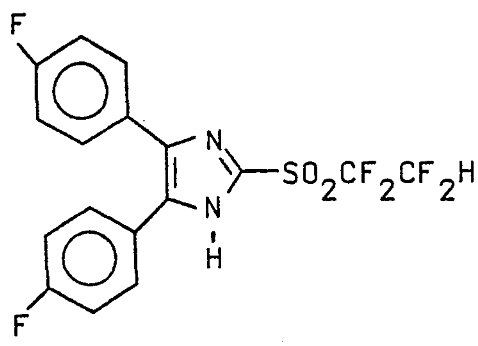

its synthesis and its use as an analgesic.

U.S. Patent 3,161,654, issued to Shen on December 15, 1964, discloses the synthesis of 1-(p-chlorobenzoyl)-5-methoxy-2-methylindole-3-acetic acid, commonly called indomethacin:

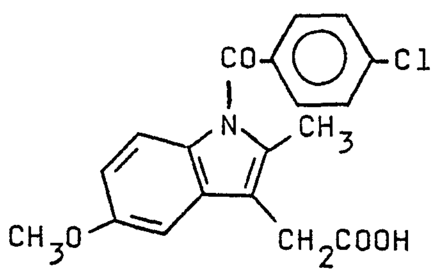

Winter et al., J. Pharmacol. Exp. Ther., 141, 369 (1963) disclose the pharmacological properties of indomethacin, and Winter and Flataker, J. Pharmacol. Exp. Ther., 150, 165 (1965) describe the analgesic properties of the compound.

U.S. Patent 4,233,313 issued to J. F. Gardocki on November 11, 1980 describes an analgesic mixture of indomethacin and acetaminophen.

U.S. Patents 3,654,349 and 3,647,858 issued to Shen et al. and Hinckley et al., respectively, disclose the synthesis and analgesic utility of (Z)-5-fluoro-2-methyl-1-{[4-(methylsulfinyl)phenyl]methylene}-1H-indene-3-acetic acid, commonly called sulindac:

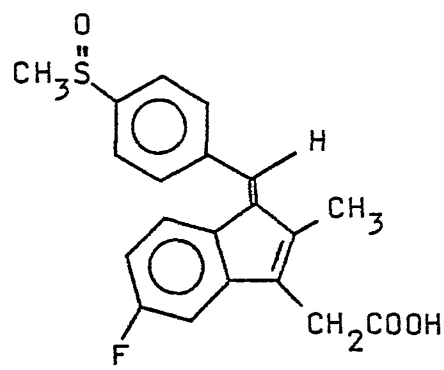

.

Van Arman et al., Fed. Proc., 31, 577 (1972) disclose the pharmacological properties and metabolism of sulindac. U.S. Patent 4,207,340 issued to J. F. Gardocki describes an analgesic mixture of sulindac and acetaminophen.

U.S. Patent 3,755,427, issued to S. S. Adams et al. on August 28, 1973, discloses the synthesis and analgesic properties of 2-fluoro-α-methyl[1,1'-biphenyl]-4-acetic acid, commonly called flurbiprofen:

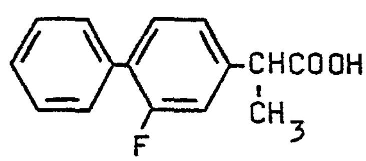

.

I. M. Chalmers et al., Ann. Rheum. Dis., 31, 19 (1972), E. M. Glenn et al., Agents and Actions, 34, 210 (1973), and R. N. Brogden et al., Drugs 18, 417 (1979) further characterize the pharmacology of flurbiprofen.

## Summary of the Invention

It has now been found that combinations of nalbuphine and certain non-narcotic analgesic compounds provide unexpectedly enhanced analgesic activity. Specifically, a pharmaceutical composition comprising a synergistically effective combination of analgesically effective amounts of nalbuphine, or a pharmaceutically suitable salt thereof, and a non-narcotic analgesic compound selected from the group consisting of ibuprofen, zomepirac, indomethacin, tiflamizole, sulindac, and flurbiprofen, or a pharmaceutically suitable salt thereof, has been found to provide enhanced pain relief in mammals. Another aspect of the invention comprises the method of alleviating pain in a mammal by administering an effective analgesic amount of the composition described above to the mammal.

## Brief Description of the Drawing

The Figures are isobologram plots characterizing effective pain relieving doses which produce analgetic responses in one half the mice subjected to the phenyl-p-benzoquinone induced writhing test at various dose ratios of nalbuphine and the indicated compound.

## Detailed Description of the Invention

Nalbuphine, which has the chemical name (-)-17-(cyclobutylmethyl)-4,5α-epoxymorphinan-3,6α,14-triol, and its preparation are described in U.S. Patent 3,393,197, the disclosure of which is hereby incorporated by reference. Ibuprofen, which has the chemical name α-methyl-4-(2-methylpropyl)benzeneacetic acid, and its preparation are described in U.S. Patents 3,228,831 and 3,385,886, the disclosures of which are hereby incorporated by reference. Zomepirac, which has the chemical name 5-(4-chlorobenzoyl)-1,4-dimethyl-1H-pyrrole-2-acetic acid, and its preparation

are described in U.S. Patent 3,752,826, the disclosure of which is hereby incorporated by reference. Indomethacin, which has the chemical name 1-(p-chlorobenzoyl)-5-methoxy-2-methylindole-3-acetic acid, and its preparation are described in U.S. Patent 3,161,654, the disclosure of which is hereby incorporated by reference. Tiflamizole, which has the chemical name 4,5-bis(4-fluorophenyl)-2-[(1,1,2,2-tetrafluoroethyl)sulfonyl]-1H-imidazole, and its preparation are described in U.S. Patent 4,190,666, the disclosure of which is hereby incorporated by reference. Sulindac, which has the chemical name (Z)-5-fluoro-2-methyl-1-{[4-(methylsulfinyl)phenyl]methylene}-1H-indene-3-acetic acid, and its preparation are described in U.S. Patents 3,654,349 and 3,647,858, the disclosures of which are hereby incorporated by reference. Flurbiprofen, which has the chemical name 2-fluoro-α-methyl[1,1'-biphenyl]-4-acetic acid, and its preparation are described in U.S. Patent 3,755,427, the disclosure of which is hereby incorporated by reference. When the terms nalbuphine, ibuprofen, zomepirac, indomethacin, tiflamizole, sulindac, and flurbiprofen are used herein, it is to be understood that any of the pharmaceutically suitable salts thereof which have analgesic properties in man and other mammals are included by the term. For nalbuphine, such salts include the hydrochloride, hydrobromide, hydroiodide, sulfate, bisulfate, nitrate, citrate, tartrate, bitartrate, phosphate, malate, maleate, fumarate, succinate, acetate, and pamoate, while for the second component, pharmaceutically suitable salts are listed in Table I.

In the composition of the invention, nalbuphine and a non-narcotic analgesic compound are combined and have been utilized at dose ratios based on weight of nalbuphine to the second component (see Table I) in

mice subjected to the phenyl-p-benzoquinone induced writhing test to establish analgetic effectiveness. The phenyl-p-benzoquinone induced writhing test in mice [H. Blumberg et al., Proc. Soc. Exp. Biol. Med., 118, 763-766 (1965)] is a standard procedure for detecting and comparing the analgetic activity of different classes of analgesic drugs with a good correlation with human analgetic activity. Data for the mouse, as presented in the isobologram, can be translated to other species where the orally effective analgesic dose of the individual compounds is known or can be estimated. The method simply consists determining the human potency ratio from the known orally effective doses of the two components, calculating the mouse potency ratio from the experimentally determined ED50 doses of the individual compounds, calculating the mouse to man scaling factor, and multiplying the ratios found effective for the mouse by the scaling factor to obtain the projected human ratios. This basic correlation for analgesic properties enables estimation of the range of human effectiveness. [E. W. Pelikan, The Pharmacologist, 1, 73 (1959).]

Application of an equieffective dose substitution model and a curvilinear regression analysis utilizing all the data for the individual compounds and various dose ratios for the combinations establishes the existence of unexpectedly enhanced analgetic activity of combinations of nalbuphine with various non-narcotic analgesic compounds, i.e., the resulting activity is greater than the activity expected from the sum of the activities of the individual components.

The composition of the invention presents the opportunity of obtaining relief from pain with reduced dosages of the two components, thereby diminishing the

side effects and toxicity which would result from the otherwise required amounts of the individual drug compounds.

Dosage Forms

The combination of analgetic agents of the invention can be administered to treat pain by any means that produces contact of the active agent with the agent's site of action in the body of a mammal. The composition of the invention can be administered by any conventional means available for use in conjunction with pharmaceuticals. It can be administered alone, but is generally administered with a pharmaceutical carrier selected on the basis of the chosen route of administration and standard pharmaceutical practice.

The dosage administered will, of course, vary depending upon known factors such as the pharmacodynamic characteristics of the particular agent, and its mode and route of administration; age, health, and weight of the recipient; nature and extent of symptoms, kind of concurrent treatment, frequency of treatment, and the effect desired. Usually a daily dosage can be such that the active ingredient is administered at a daily dosage of from about 0.25 to 7.50 milligrams per kilogram (mg/kg) of body weight of nalbuphine and an amount for the second component as listed in Table I. Ordinarily, administration of the composition of the invention in divided doses 2-5 times a day or in a sustained release form is effective to obtain desired results.

Dosage forms (compositions) suitable for internal administration contain a total of from about 15 milligrams to about 600 milligrams of active ingredients per unit. In these pharmaceutical compositions the active ingredients will ordinarily be present in

an amount of about 0.5-95% by weight based on the total weight of the composition.

The active ingredients can be administered orally in solid dosage forms, such as capsules, tablets, and powders, or in liquid dosage forms, such as elixirs, syrups, and suspensions.

Gelatin capsules contain the active ingredients and powdered carriers, such as lactose, sucrose, mannitol, starch, cellulose derivatives, magnesium stearate, stearic acid, and the like. Suitable vehicles, excipients and co-helpers are available to formulate the compositions of this invention. Similar carriers, vehicles, excipients and co-helpers can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

Suitable pharmaceutical carriers are described in Remington's Pharmaceutical Sciences, A. Osol, a standard reference text in this field.

Useful pharmaceutical dosage-forms for administration of the composition of the invention can be illustrated by the following examples:

Example 1

Nalbuphine/Ibuprofen Tablets (30/60 mg)

| Formula | mg/Tablet |
|---|---|
| Nalbuphine HCl | 30.0 |
| Ibuprofen | 60.0 |
| Microcrystalline Cellulose | 140.0 |
| Starch, modified | 16.0 |
| Stearic Acid | 4.0 |
| | 250.0 |

Example 2

Nalbuphine/Ibuprofen Tablets (30/300 mg)

| Formula | mg/Tablet |
|---|---|
| Nalbuphine HCl | 30.0 |
| Ibuprofen | 300.0 |
| Microcrystalline Cellulose | 190.0 |
| Starch, modified | 22.0 |
| Stearic Acid | 8.0 |
| | 550.0 |

Example 3

Nalbuphine/Ibuprofen Tablets (7.5/300 mg)

| Formula | mg/Tablet |
|---|---|
| Nalbuphine HCl | 7.5 |
| Ibuprofen | 300.0 |
| Microcrystalline Cellulose | 212.5 |
| Starch, modified | 22.0 |
| Stearic Acid | 8.0 |
| | 550.0 |

## Example 4

Nalbuphine/Ibuprofen Capsules (30/60 mg)

| Formula | mg/Capsule |
|---|---|
| Nalbuphine HCl | 30.0 |
| Ibuprofen | 60.0 |
| Microcrystalline Cellulose | 140.0 |
| Starch, modified | 112.0 |
| Starch | 8.0 |
| | 350.0 |

## Example 5

Nalbuphine/Ibuprofen Capsules (30/300 mg)

| Formula | mg/Capsule |
|---|---|
| Nalbuphine HCl | 30.0 |
| Ibuprofen | 300.0 |
| Microcrystalline Cellulose | 90.0 |
| Starch; modified | 7.0 |
| Starch | 8.0 |
| | 435.0 |

## Example 6

Nalbuphine/Ibuprofen Capsules (7.5/300 mg)

| Formula | mg/Capsule |
|---|---|
| Nalbuphine HCl | 7.5 |
| Ibuprofen | 300.0 |
| Microcrystalline Cellulose | 110.0 |
| Starch, modified | 9.5 |
| Starch | 8.0 |
| | 435.0 |

Example 7

Nalbuphine/Zomepirac Tablets (30/6 mg)

| Formula | mg/Tablet |
|---|---|
| Nalbuphine HCl | 30.0 |
| Zomepirac Sodium | 6.0 |
| Microcrystalline Cellulose | 190.0 |
| Starch, modified | 22.0 |
| Magnesium Stearate | 2.0 |
| | 250.0 |

Example 8

Nalbuphine/Zomepirac Tablets (30/50 mg)

| Formula | mg/Tablet |
|---|---|
| Nalbuphine HCl | 30.0 |
| Zomepirac Sodium | 50.0 |
| Microcrystalline Cellulose | 240.0 |
| Starch, modified | 27.0 |
| Magnesium Stearate | 3.0 |
| | 350.0 |

Example 9

Nalbuphine/Zomepirac Tablets (15/150 mg)

| Formula | mg/Tablet |
|---|---|
| Nalbuphine HCl | 15.0 |
| Zomepirac Sodium | 150.0 |
| Microcrystalline Cellulose | 210.0 |
| Starch, modified | 21.0 |
| Magnesium Stearate | 4.0 |
| | 400.0 |

### Example 10

Nalbuphine/Zomepirac Capsules (30/6 mg)

| Formula | mg/Capsule |
|---|---|
| Nalbuphine HCl | 30.0 |
| Zomepirac Sodium | 6.0 |
| Microcrystalline Cellulose | 190.0 |
| Starch, modified | 20.0 |
| Starch | 4.0 |
| | 250.0 |

### Example 11

Nalbuphine/Zomepirac Capsules (30/50 mg)

| Formula | mg/Capsule |
|---|---|
| Nalbuphine HCl | 30.0 |
| Zomepirac Sodium | 50.0 |
| Microcrystalline Cellulose | 240.0 |
| Starch, modified | 23.0 |
| Starch | 7.0 |
| | 350.0 |

### Example 12

Nalbuphine/Zomepirac Capsules (15/150 mg)

| Formula | mg/Capsule |
|---|---|
| Nalbuphine HCl | 15.0 |
| Zomepirac Sodium | 150.0 |
| Microcrystalline Cellulose | 205.0 |
| Starch, modified | 21.0 |
| Starch | 9.0 |
| | 400.0 |

Example 13

Nalbuphine/Indomethacin Tablets (30/12 mg)

| Formula | mg/Tablet |
|---|---|
| Nalbuphine HCl | 30.0 |
| Indomethacin | 12.0 |
| Microcrystalline Cellulose | 184.0 |
| Starch, modified | 20.0 |
| Stearic Acid | 4.0 |
| | 250.0 |

Example 14

Nalbuphine/Indomethacin Tablets (30/25 mg)

| Formula | mg/Tablet |
|---|---|
| Nalbuphine HCl | 30.0 |
| Indomethacin | 25.0 |
| Microcrystalline Cellulose | 220.0 |
| Starch, modified | 20.0 |
| Stearic Acid | 5.0 |
| | 300.0 |

Example 15

Nalbuphine/Indomethacin Tablets (15/150 mg)

| Formula | mg/Tablet |
|---|---|
| Nalbuphine HCl | 15.0 |
| Indomethacin | 150.0 |
| Microcrystalline Cellulose | 208.0 |
| Starch, modified | 20.0 |
| Stearic Acid | 7.0 |
| | 400.0 |

Example 16

Nalbuphine/Indomethacin Capsules (30/12 mg)

| Formula | mg/Capsule |
|---|---|
| Nalbuphine HCl | 30.0 |
| Indomethacin | 12.0 |
| Microcrystalline Cellulose | 275.0 |
| Starch, modified | 25.0 |
| Starch | 8.0 |
| | 350.0 |

Example 17

Nalbuphine/Indomethacin Capsules (30/25 mg)

| Formula | mg/Capsule |
|---|---|
| Nalbuphine HCl | 30.0 |
| Indomethacin | 25.0 |
| Microcrystalline Cellulose | 260.0 |
| Starch, modified | 27.0 |
| Starch | 8.0 |
| | 350.0 |

Example 18

Nalbuphine/Indomethacin Capsules (15/150 mg)

| Formula | mg/Capsule |
|---|---|
| Nalbuphine HCl | 15.0 |
| Indomethacin | 150.0 |
| Microcrystalline Cellulose | 160.0 |
| Starch, modified | 17.0 |
| Starch | 8.0 |
| | 350.0 |

Example 19

Nalbuphine/Tiflamizole Tablets (30/0.15 mg)

| Formula | mg/Tablet |
|---|---|
| Nalbuphine HCl | 30.00 |
| Tiflamizole | 0.15 |
| Microcrystalline Cellulose | 200.00 |
| Starch, modified | 17.35 |
| Magnesium Stearate | 2.50 |
| | 250.00 |

Example 20

Nalbuphine/Tiflamizole Tablets (30/0.5 mg)

| Formula | mg/Tablet |
|---|---|
| Nalbuphine HCl | 30.0 |
| Tiflamizole | 0.5 |
| Microcrystalline Cellulose | 200.0 |
| Starch, modified | 17.0 |
| Magnesium Stearate | 2.5 |
| | 250.0 |

Example 21

Nalbuphine/Tiflamizole Tablets (30/3.0 mg)

| Formula | mg/Tablet |
|---|---|
| Nalbuphine HCl | 30.0 |
| Tiflamizole | 3.0 |
| Microcrystalline Cellulose | 197.5 |
| Starch, modified | 17.0 |
| Magnesium Stearate | 2.5 |
| | 250.0 |

Example 22

Nalbuphine/Tiflamizole Capsules (30/0.15 mg)

| Formula | mg/Capsule |
|---|---|
| Nalbuphine HCl | 30.00 |
| Tiflamizole | 0.15 |
| Microcrystalline Cellulose | 280.00 |
| Starch, modified | 31.85 |
| Starch | 8.00 |
| | 350.00 |

Example 23

Nalbuphine/Tiflamizole Capsules (30/0.5 mg)

| Formula | mg/Capsule |
|---|---|
| Nalbuphine HCl | 30.0 |
| Tiflamizole | 0.5 |
| Microcrystalline Cellulose | 280.0 |
| Starch, modified | 31.5 |
| Starch | 8.0 |
| | 350.0 |

Example 24

Nalbuphine/Tiflamizole Capsules (30/3.0 mg)

| Formula | mg/Capsule |
|---|---|
| Nalbuphine HCl | 30.0 |
| Tiflamizole | 3.0 |
| Microcrystalline Cellulose | 280.0 |
| Starch, modified | 29.0 |
| Starch | 8.0 |
| | 350.0 |

Example 25

Nalbuphine/Sulindac Tablets (30/30 mg)

| Formula | mg/Tablet |
|---|---|
| Nalbuphine HCl | 30.0 |
| Sulindac | 30.0 |
| Microcrystalline Cellulose | 170.0 |
| Starch, modified | 16.0 |
| Stearic Acid | 4.0 |
| | 250.0 |

Example 26

Nalbuphine/Sulindac Tablets (30/150 mg)

| Formula | mg/Tablet |
|---|---|
| Nalbuphine HCl | 30.0 |
| Sulindac | 150.0 |
| Microcrystalline Cellulose | 150.0 |
| Starch, modified | 14.0 |
| Stearic Acid | 6.0 |
| | 350.0 |

Example 27

Nalbuphine/Sulindac Tablets (7.5/300 mg)

| Formula | mg/Tablet |
|---|---|
| Nalbuphine HCl | 7.5 |
| Sulindac | 300.0 |
| Microcrystalline Cellulose | 212.5 |
| Starch, modified | 22.0 |
| Stearic Acid | 8.0 |
| | 550.0 |

Example 28

Nalbuphine/Sulindac Capsules (30/30 mg)

| Formula | mg/Capsule |
|---|---|
| Nalbuphine HCl | 30.0 |
| Sulindac | 30.0 |
| Microcrystalline Cellulose | 170.0 |
| Starch, modified | 112.0 |
| Starch | 8.0 |
| | 350.0 |

Example 29

Nalbuphine/Sulindac Capsules (30/150 mg)

| Formula | mg/Capsule |
|---|---|
| Nalbuphine HCl | 30.0 |
| Sulindac | 150.0 |
| Microcrystalline Cellulose | 150.0 |
| Starch, modified | 12.0 |
| Starch | 8.0 |
| | 350.0 |

Example 30

Nalbuphine/Sulindac Capsules (7.5/300 mg)

| Formula | mg/Capsule |
|---|---|
| Nalbuphine HCl | 7.5 |
| Sulindac | 300.0 |
| Microcrystalline Cellulose | 110.0 |
| Starch, modified | 9.5 |
| Starch | 8.0 |
| | 435.0 |

Example 31

Nalbuphine/Flurbiprofen Tablets (30/30 mg)

| Formula | mg/Tablet |
|---|---|
| Nalbuphine HCl | 30.0 |
| Flurbiprofen | 30.0 |
| Microcrystalline Cellulose | 210.0 |
| Starch, modified | 25.0 |
| Stearic Acid | 5.0 |
| | 300.0 |

Example 32

Nalbuphine/Flurbiprofen Tablets (30/100 mg)

| Formula | mg/Tablet |
|---|---|
| Nalbuphine HCl | 30.0 |
| Flurbiprofen | 100.0 |
| Microcrystalline Cellulose | 240.0 |
| Starch, modified | 23.0 |
| Stearic Acid | 7.0 |
| | 400.0 |

Example 33

Nalbuphine/Flurbiprofen Tablets (15/200 mg)

| Formula | mg/Tablet |
|---|---|
| Nalbuphine HCl | 15.0 |
| Flurbiprofen | 200.0 |
| Microcrystalline Cellulose | 160.0 |
| Starch, modified | 17.0 |
| Stearic Acid | 8.0 |
| | 400.0 |

Example 34

Nalbuphine/Flurbiprofen Capsules (30/30 mg)

| Formula | mg/Capsule |
|---|---|
| Nalbuphine HCl | 30.0 |
| Flurbiprofen | 30.0 |
| Microcrystalline Cellulose | 260.0 |
| Starch, modified | 22.0 |
| Starch | 8.0 |
| | 350.0 |

Example 35

Nalbuphine/Flurbiprofen Capsules (30/100 mg)

| Formula | mg/Capsule |
|---|---|
| Nalbuphine HCl | 30.0 |
| Flurbiprofen | 100.0 |
| Microcrystalline Cellulose | 190.0 |
| Starch, modified | 22.0 |
| Starch | 8.0 |
| | 350.0 |

Example 36

Nalbuphine/Flurbiprofen Capsules (15/200 mg)

| Formula | mg/Capsule |
|---|---|
| Nalbuphine HCl | 15.0 |
| Flurbiprofen | 200.0 |
| Microcrystalline Cellulose | 110.0 |
| Starch, modified | 17.0 |
| Starch | 8.0 |
| | 350.0 |

Test Methods

The unexpectedly enhanced analgetic activity obtained in the method of the invention is evidenced by tests conducted on mice. Male $CF_1$ mice obtained from Charles River Breeding Laboratories, fasted for 16-22 hours and weighing 18-22g at the time of testing are used throughout. All mice are dosed sequentially by the oral route with suspensions or solutions of the compounds. A dosing volume of 10 ml/kg is used for each sequential solution or suspension. It will be appreciated by those skilled in the art that the enhanced activity will be obtained whether the compounds are administered simultaneously as a mixture or sequentially as the two individual components. All doses are coded and the test is performed under a code not known to the observer.

Stock solutions of nalbuphine HCl are prepared by dissolving dry nalbuphine hydrochloride powder with distilled water. All dosing solutions are prepared by dilution of the stock solution with distilled water; the vehicle control is distilled water.

A stock suspension of ibuprofen is prepared by mixing 143.7 mg ibuprofen with 40 ml of an aqueous vehicle containing 2% by volume of Tween 80®, a pharmacological dispersant manufactured by Fisher Scientific Company and containing 100% polysorbate 80, and 1% by weight of Methocel® MC powder, a suspending agent manufactured by DOW Chemical Company and containing 100% methylcellulose, in distilled water. The mixture is sonicated at 150 watts for 1-2 minutes with an ultrasound system, then shaken for two hours at 280 oscillations/minute with 15-20 gm of glass beads. The resultant suspension contains 3.59 mg/ml of ibuprofen; all dosing suspensions are prepared by dilution of the stock suspension with the Methocel®/Tween 80® vehicle; the vehicle control is Methocel®/Tween 80®.

A stock solution of zomepirac is prepared by dissolving dry zomepirac sodium powder with an aqueous vehicle containing 2% by volume of Tween 80®. All dosing solutions are prepared by dilution of the stock solution with the 2% Tween 80® vehicle; the vehicle control is 2% Tween 80®.

A stock solution of indomethacin is prepared by dissolving dry indomethacin powder with an aqueous vehicle containing 2% by volume of Tween 80®. All dosing solutions are prepared by dilution of the stock solution with the 2% Tween 80® vehicle; the vehicle control is 2% Tween 80®.

A stock suspension of tiflamizole is prepared by mixing 35.88 mg tiflamizole with 40 ml of an aqueous vehicle containing 2% by volume of Tween 80®. The mixture is sonicated at 150 watts for 1-2 minutes with an ultrasound system, then shaken for two hours at 280 oscillations/minute with 15-20 gm of glass beads. The resultant suspension contains 0.897 mg/ml of tiflamizole; all dosing suspensions are prepared by dilution of the stock suspension with the Methocel®/Tween 80® vehicle; the vehicle control is Methocel®/Tween 80®.

A stock suspension of sulindac is prepared by mixing 800 mg sulindac with 50 ml of an aqueous vehicle containing 2% by volume of Tween 80®. The mixture is sonicated at 150 watts for 1-2 minutes with an ultrasound system, then shaken for two hours at 280 oscillations/minute with 15-20 gm of glass beads. The resultant suspension contains 16 mg/ml of sulindac; all dosing suspensions are prepared by dilution of the stock suspension with the Methocel®/Tween 80® vehicle; the vehicle control is Methocel®/Tween 80®.

A stock solution of flurbiprofen is prepared by dissolving dry flurbiprofen powder with an equimolar amount of one molar sodium hydroxide and sufficient

water to produce the required solution concentration. The acidity is adjusted with dilute hydrochloric acid to pH 9.0. All dosing solutions are prepared by dilution of the stock solution with distilled water; the vehicle control is distilled water.

All dosing solutions and suspensions were prepared fresh daily.

As indicated above, the standard procedure based upon the prevention of phenyl-p-benzoquinone induced writhing in mice is utilized to detect and quantify the analgetic activity of the compositions of this invention.

Mice, intubated with various doses of nalbuphine hydrochloride, the second non-narcotic analgesic compound, combined doses of nalbuphine hydrochloride and the second component, or vehicle, are injected intraperitoneally with a challenge dose of phenyl-p-benzoquinone 5 minutes prior to the designated observation period. The phenyl-p-benzoquinone is prepared as an 0.1 mg/ml solution in 5% by volume of ethanol in water; the writhing dose is 1.25 mg/kg injected in a volume of 0.25 ml/20g. For scoring purposes a "writhe" is indicated by whole body stretching or contraction of the abdomen; mice are observed 10 minutes for the presence or absence of writhing beginning 5 minutes after receiving the phenyl-p-benzoquinone dose. Each mouse is used only once, then discarded. The alleviation of pain is quantified by determining the dosage at which 50% of the mice in a test group exhibit an analgesic response for the composition being tested. This dosage as described herein is referred to as the ED50. All ED50 values and their 95% confidence limits are determined numerically by the computer-assisted methods of Finney [D. J. Finney, "Probit Analysis", Third Edition, Cambridge University Press, Cambridge, England, 1971].

In order to study the interaction between nalbuphine and the second component, a number of precise dosage ratios are selected. Four or five coded doses of each selected combination are studied for analgesic effectiveness at times varying from 5 to 80 minutes using an experimental design which permits coding and complete randomization of the separate dosage forms tested. The times, number of dosage forms tested, and specific dose ratios utilized are shown in Tables II through VII.

The nature of the analgetic interaction (addition, synergism, or antagonism) is determined by graphing the results in a Loewe isobologram [S. Loewe, Pharm. Rev. 9:237-242 (1957)]. The isobologram is a quantitative method for measuring interactions between drugs where dose-effect relationships are depicted in a multi-dimensional array with lines connecting dose pairs that are equieffective in relationship to a common pharmacological endpoint. In this instance, the antiphenylquinone writhing test is used to estimate a common level of analgesic activity (ED50 dose) for the two component drugs separately and for each fixed dose-ratio combination. In the isobolographic figure, areas of dose addition, synergism, and/or antagonism are clearly defined by reference to the theoretical "ED50 Addition Line." According to Loewe's isobolographic theory, ED50's falling under the curve (between the ED50 Addition Line and the origin) would represent unexpectedly enhanced analgetic activity and combination ED50's located above the line would represent unexpectedly diminished analgetic activity.

Most importantly, the isobolographic technique permits a full range of doses and dose combinations to be examined where the proportion of the first drug to the second actually varies from 0 to infinity, and to

determine, by virtue of the graphical display, whether any one or more of the paired drug combinations displays unique pharmacological properties in comparison to the entire body of data generated. The isobologram is also valuable for organizing the data in a form which is easily amenable to statistical assessment of observed differences.

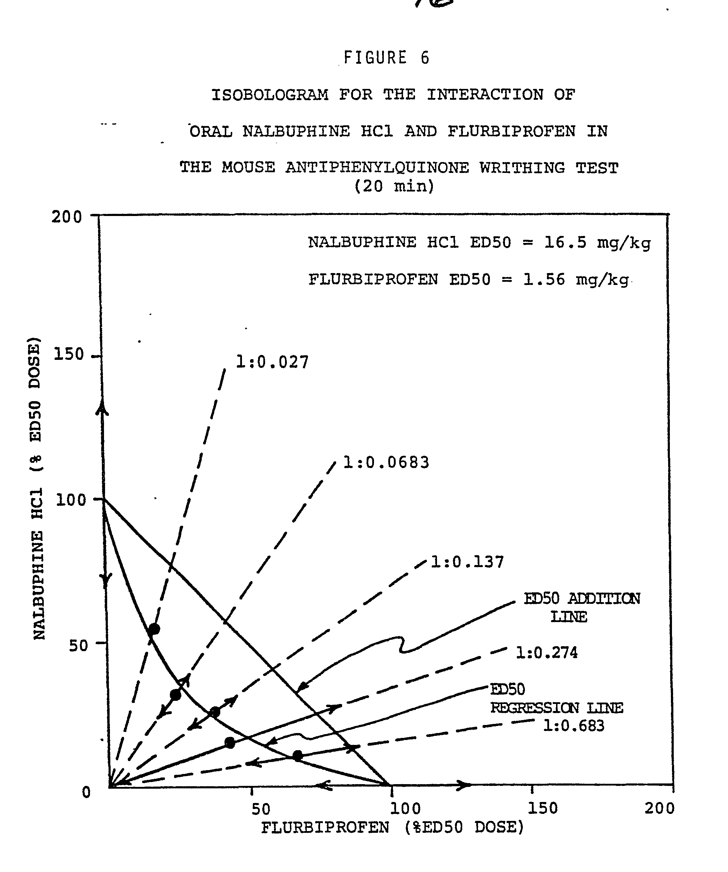

The synergistic interaction of nalbuphine hydrochloride and the second component on phenyl-p-benzoquinone induced writhing in mice is demonstrated by the data in Tables II through VII and in Figures 1 through 6, the Loewe isobolograms. In such isobolographic figures, the analgetic effect of nalbuphine alone is presented in the ordinate, and that of the second component alone is on the abscissa. The dotted lines radiating from the origin represent the exact fixed dosage ratios based on weight of nalbuphine HCl:the second component in the ranges indicated. ED50 values are marked on the ordinate and abscissa, representing nalbuphine and the second component alone, and on the dotted radial lines, representing the compositions of nalbuphine and the second component at the fixed dosage ratios. The arrows extending above and below each ED50 point represent the 95% confidence limits of the ED50's.

As drawn in the Figures, the solid diagonal line joining the ED50 values of the two drugs given separately represents the "ED50 Addition Line," the theoretical line for simple additivity of drug effects which would be observed in the absence of a synergistic response. The drawing clearly shows that in the method of the invention, all of the tested fixed ratio compositions give unexpectedly enhanced analgetic activity since the ED50 values for each of these ratios fall below the line of simple additivity.

By utilizing an equieffective dose substitution model and a statistical regression analysis of all of the data, one can obtain a more reliable assessment of the existence of a synergistic property, in this case unexpectedly enhanced analgetic activity. The effects of two compounds are additive if the response to a dose of the two in combination does not change when a portion of one is removed from the mixture and replaced by an equipotent portion of the other. If such substitution increases the response, the mixing together of the compounds is said to potentiate their effects and synergism exists.

Consider ED50 doses of mixtures of X units of compound B with Y units of compound A, whose ED50 doses are $\beta$ and $\alpha$, respectively. Given the hypothesis of additivity, all doses of mixtures satisfying the straight line relation,

$$(1) \qquad Y = \alpha - \frac{\alpha}{\beta} X ,$$

will be ED50 doses. To test the hypothesis of additivity, ED50 doses of mixtures are estimated through probit analysis of data from experiments run at various ratios of A to B. Linear and curvilinear regression models are fitted to the data to estimate the amounts of A in respective ED50 doses, given the amount of B, (or, conversely, the amount of B, given A). If a curvilinear regression fit the data significantly better than a straight line regression, the hypothesis of additivity is refuted and synergism exists for the two compounds for the property of interest.

Values of Y calculated from the straight line of Equation 1, and values of Y calculated from the curvi-

linear regression are plotted against X on an ED50 isobologram to describe the synergism.

It is convenient to standardize the units of dose such that 100 units of either compound alone is its respective estimated ED50 dose. The additivity hypothesis, then, will be represented by a straight line from 100 on the Y-axis to 100 on the X-axis on the isobologram, and Equation (1) becomes:

$$Y = 100 - X.$$

The isobolograms contained in Figures 1-6 show the straight line additivity hypothesis for nalbuphine HCl and the indicated second component at the time specified post oral dosing in the mouse antiphenylquinone writhing test. Data were standardized to the ED50 doses of nalbuphine HCl and the second component as indicated in the legend for each Figure. Synergism is demonstrated in all examples by the regression fitted to ED50 dose levels estimated by probit analysis. Its curvilinearity is statistically significant.

The regression is fitted to the data by the method of least squares. Residual squared deviations about the line of best fit are minimized in directions along lines from the origin through respective data points on the isobologram, these lines making angles with X-axis, $\tan^{-1}(Y/X)$. This is accomplished by a transformation prior to the regression analysis. Its inverse is applied to transform the coordinates of the regression curve back to the X,Y coordinates of the isobologram.

Let $D_r$ be an ED50 dose of a mixture of A and B, where r is the fraction of compound B in the mixture; i.e.

$$r = \frac{X}{X + Y}.$$

It follows from Equation 1 that

$$D_r = \frac{\alpha\beta}{\alpha r + \beta(1-r)}.$$

From the additivity hypothesis, the logarithms of the ED50 doses at various mixture ratios are a straight line function of (Log $D_r$). To test the hypothesis, polynomial regressions, as follows, are fitted to ED50 estimates from experimental data obtained at various mixture ratios:

$$(2) \quad F_r = \log D_r = b_0 + \sum_{i=1}^{K} b_i \left\{ \log \left[ \frac{\alpha\beta}{\alpha r + \beta(1-r)} \right] \right\}^i$$

The additivity hypothesis is refuted if a polynomial of degree higher than one fit the data significantly better than a straight line,

$$F_r = b_0 + b_1 \left[ \log \left( \frac{\alpha\beta}{\alpha r + \beta(1-r)} \right) \right]$$

Since X and Y are uniquely determined by $F_r$ and r, the coordinates of the regression are transformed readily to the coordinates of the isobologram.

If data are scaled to ED50 dose levels of 100 standard dose units, Equation (2) becomes

$$(2.1) \quad F_s = \log 100 = 2.$$

The additivity hypothesis implies that $F_s$ is independent of $r_s$, and may be tested by analysis of the regression model

$$(2.2) \quad F_s = b_0 + \sum_{i=1}^{K} b_i r_s^{\,i},$$

the subscripts, s, indicating that the data are scaled. A statistically significant regression will refute the hypothesis.

The method of least squares utilizes jointly the information contained in all of the separate data points. Statistical significance of the curvilinearity of the regression model establishes the existence of synergism (or antagonism) of the compounds in the biological system studied. The parameters in the model describe its intensity over the range of mixture ratios, from 0 to 1, the nature of which is seen readily when the regression is plotted on the isobologram. This method was used to determine the best-fitting ED50 regression line through the ED50 data points representing equivalent levels of analgetic activity for each of the dose-ratio compositions and for nalbuphine and the second component alone as listed in Tables II-VII. As shown in the isobologram Figures, the calculated quadratic polynomial "ED50 Regression Line" fits the data significantly better than the straight "ED50 Addition Line" as established by Fisher's F test (statistically significant at values of P ranging from $P \leq 0.003$ to $P \leq 0.05$) to compare the goodness of fit between the straight line and the curvilinear regression. Thus, consistent with Loewe's isobolographic model, the hypothesis of analgetic additivity is refuted and analgetic synergism is established for all combinations of nalbuphine and the second component of each mixture.

By substitution of the expected analgetic activity of nalbuphine alone and the second component alone from test results in other warm blooded mammals, it is possible to use the isobologram in conjunction with the correlation method discussed above to predict the equivalent range of maximum potentiating dosages for

man. Thus utilizing the data of the present invention and the equivalent ratios in man, it is predicted that the compositions of the instant invention would demonstrate analgetic potentiation over a range of doses as shown in Table I.

As described above, all tests of statistical significance establishing the best fit regression equation for the experimental data and its difference from the ED50 Addition Line were carried out using stringent 95% confidence limits. The use of less stringent limits merely reinforces the conclusions.

It will be apparent that the instant specification and examples are set forth by way of illustration and not limitation, and that various modifications and changes may be made without departing from the spirit and scope of the present invention.

TABLE I



| Non-narcotic Analgesic Compound | Pharmaceutically Suitable Salts | Range of Dose Ratios Experimentally Tested |
|---|---|---|
| Ibuprofen | Al,Ca,K,Na | 1:0.24 to 1:5.89 |
| Zomepirac | Ca,K,Na | 1:0.021 to 1:1.5 |
| Indomethacin | Ca,K,Na | 1:0.0146 to 1:0.366 |
| Tiflamizole | Ca,K,Na | 1:0.0246 to 1:0.617 |
| Sulindac | Ca,K,Na | 1:0.175 to 1:4.37 |
| Flurbiprofen | Al,Ca,K,Na | 1:0.027 to 1:0.683 |

| Non-narcotic Analgesic Compound | Daily Dosage Range (mg/kg) | Range of Human Analgesic Potentiation |
|---|---|---|
| Ibuprofen | 10-120 | 1:0.40 to 1:79 |
| Zomepirac | 1-20 | 1:0.02 to 1:26 |
| Indomethacin | 0.5-5.0 | 1:0.01 to 1:20 |
| Tiflamizole | 0.002-0.32 | 1:0.0046 to 1:0.62 |
| Sulindac | 1.0-13 | 1:0.1 to 1:400 |
| Flurbiprofen | 1-10 | 1:0.027 to 1:15 |

# TABLE II

ORAL NALBUPHINE/IBUPROFEN COMBINATIONS IN THE MOUSE ANTIPHENYLQUINONE WRITHING TEST

5 Min (N=30-70 Mice/Dose)

| DRUG COMBINATIONS Nalbuphine HCl: Ibuprofen | DRUG DOSE (mg/kg) | | % MICE BLOCKED | ED50 AT 5 MIN (95% Confidence Limits) | |
|---|---|---|---|---|---|
| | Nalbuphine HCl | Ibuprofen | | Nalbuphine HCl | Ibuprofen |
| Control (0:0) | 0 | 0 | 14.3% | - | - |
| Nalbuphine Only (1:0) | 1.9<br>3.8<br>7.6<br>15.2<br>30.4 | 0<br>0<br>0<br>0<br>0 | 12.9%<br>25.7%<br>50.0%<br>80.0%<br>88.6% | 9.12<br>(7.04-11.2) | 0.0 |
| 1:0.24 | 1.58<br>3.17<br>6.33<br>12.7<br>25.3 | 0.37<br>0.75<br>1.50<br>2.99<br>5.99 | 33.3%<br>30.0%<br>46.7%<br>93.3%<br>96.7% | 6.82<br>(4.65-8.77) | 1.61<br>(1.10-2.08) |
| 1:0.59 | 1.27<br>2.54<br>5.07<br>10.1<br>20.3 | 0.75<br>1.50<br>2.99<br>5.99<br>12.0 | 6.7%<br>40.0%<br>63.3%<br>76.7%<br>100.0% | 4.62<br>(3.18-6.07) | 2.72<br>(1.87-3.58) |
| 1:1.18 | 0.95<br>1.9<br>3.8<br>7.6<br>15.2 | 1.12<br>2.25<br>4.49<br>8.98<br>18.0 | 24.3%<br>35.7%<br>64.3%<br>92.9%<br>100.0% | 3.22<br>(2.54-3.83) | 3.80<br>(3.00-4.53) |
| 1:2.37 | 0.63<br>1.27<br>2.53<br>5.06<br>10.1 | 1.50<br>2.99<br>5.99<br>12.0<br>24.0 | 10.0%<br>36.7%<br>50.0%<br>80.0%<br>100.0% | 2.63<br>(1.83-3.40) | 6.21<br>(4.33-8.03) |
| 1:5.89 | 0.32<br>0.64<br>1.27<br>2.54<br>5.08 | 1.87<br>3.74<br>7.48<br>15.0<br>29.9 | 20.0%<br>40.0%<br>50.0%<br>80.0%<br>100.0% | 1.26<br>(0.86-1.66) | 7.44<br>(5.05-9.79) |
| Ibuprofen Only (0:1) | 0<br>0<br>0<br>0<br>0 | 2.25<br>4.49<br>8.98<br>18.0<br>35.9 | 18.6%<br>24.3%<br>41.4%<br>67.1%<br>92.5% | 0.0 | 13.6<br>(10.5-16.7) |

# TABLE III

**ORAL NALBUPHINE/ZOMEPIRAC SODIUM COMBINATIONS IN THE MOUSE ANTIPHENYLQUINONE WRITHING TEST**

**20 Min (N=45 Mice/Dose)**

| DRUG COMBINATIONS Nalbuphine HCl: Zomepirac Na | DRUG DOSE (mg/kg) | | % MICE BLOCKED | ED50 At 20 MIN (95% Confidence Limits) | |
|---|---|---|---|---|---|
| | Nalbuphine HCl | Zomepirac Sodium | | Nalbuphine HCl | Zomepirac Sodium |
| Control (0:) | 0 | 0 | 6.7% | - | - |
| Nalbuphine Only (1:0) | 2.75<br>5.5<br>11<br>22<br>44 | 0<br>0<br>0<br>0<br>0 | 15.6%<br>24.4%<br>62.2%<br>68.9%<br>84.4% | 12.0<br>(8.70-15.8) | 0.0 |
| 1:0.021 | 2.29<br>4.58<br>9.17<br>18.3<br>36.7 | 0.048<br>0.097<br>0.193<br>0.387<br>0.773 | 13.3%<br>26.7%<br>66.7%<br>93.3%<br>100.0% | 7.41<br>(5.88-8.87) | 0.152<br>(0.120-0.81) |
| 1:0.053 | 1.83<br>3.67<br>7.33<br>14.7<br>29.3 | 0.097<br>0.193<br>0.387<br>0.733<br>1.55 | 17.8%<br>35.6%<br>62.2%<br>95.6%<br>100.0% | 5.46<br>(4.23-6.65) | 0.288<br>(0.223-0.351) |
| 1:0.105 | 1.38<br>2.75<br>5.5<br>11<br>22 | 0.145<br>0.29<br>0.58<br>1.16<br>2.32 | 6.7%<br>26.7%<br>66.7%<br>93.3%<br>100.0% | 4.05<br>(3.26-4.81) | 0.426<br>(0.344-0.506) |
| 1:0.210 | 0.917<br>1.83<br>3.67<br>7.33<br>14.7 | 0.193<br>0.387<br>0.733<br>1.55<br>3.09 | 15.6%<br>28.9%<br>62.2%<br>91.1%<br>97.8% | 2.99<br>(2.31-3.67) | 0.627<br>(0.485-0.770) |
| 1:0.528 | 0.458<br>0.917<br>1.83<br>3.67<br>7.33 | 0.242<br>0.483<br>0.967<br>1.93<br>3.87 | 11.1%<br>26.7%<br>62.2%<br>88.9%<br>97.8% | 1.58<br>(1.23-1.92) | 0.832<br>(0.652-1.01) |
| 1:1.5 | 0.275<br>0.55<br>1.10<br>2.20<br>4.40 | 0.413<br>0.825<br>1.65<br>3.30<br>6.60 | 20.0%<br>31.1%<br>60.0%<br>95.6%<br>95.6% | 0.859<br>(0.65-1.06) | 1.28<br>(0.977-1.60) |
| Zomepirac Sodium Only (0:1) | 0<br>0<br>0<br>0 | 0.29<br>0.58<br>1.16<br>2.32 | 13.3%<br>20.0%<br>31.1%<br>75.6% | 0.0 | 1.66<br>(1.29-2.09) |

# TABLE IV

ORAL NALBUPHINE/INDOMETHACIN COMBINATIONS IN THE MOUSE ANTIPHENYLQUINONE WRITHING TEST

40 Min (N=60 Mice/Dose)

| DRUG COMBINATIONS Nalbuphine HCl: Indomethacin | DRUG DOSE (mg/kg) | | % MICE BLOCKED | ED50 AT 40 MIN (95% Confidence Limits) | |
|---|---|---|---|---|---|
| | Nalbuphine HCl | Indomethacin | | Nalbuphine HCl | Indomethacin |
| Control (0:0) | 0 | 0 | 3.3% | - | - |
| Nalbuphine Only (1:0) | 5.45<br>10.9<br>21.8<br>43.6<br>87.2 | 0<br>0<br>0<br>0<br>0 | 10.0%<br>13.3%<br>46.7%<br>66.7%<br>66.7% | 35.5<br>(13.7-126) | 0.0 |
| 1:0.0146 | 4.54<br>9.08<br>18.2<br>36.3<br>72.7 | 0.066<br>0.133<br>0.265<br>0.530<br>1.06 | 13.3%<br>30.0%<br>61.7%<br>96.7%<br>100.0% | 13.7<br>(11.4-16.0) | 0.199<br>(0.166-0.232) |
| 1:0.0365 | 3.63<br>7.27<br>14.5<br>29.1<br>58.1 | 0.133<br>0.265<br>0.530<br>1.06<br>2.12 | 18.3%<br>41.7%<br>80.0%<br>100.0%<br>100.0% | 8.06<br>(6.81-9.34) | 0.294<br>(0.248-0.340) |
| 1:0.0729 | 2.73<br>5.45<br>10.9<br>21.8<br>43.6 | 0.199<br>0.398<br>0.795<br>1.59<br>3.18 | 25.0%<br>55.0%<br>83.3%<br>100.0%<br>100.0% | 5.00<br>(4.15-5.84) | 0.363<br>(0.303-0.424) |
| 1:0.146 | 1.82<br>3.63<br>7.27<br>14.5<br>29.1 | 0.199<br>0.530<br>1.06<br>2.12<br>4.24 | 25.0%<br>46.7%<br>91.7%<br>100.0%<br>98.3% | 3.33<br>(0.534-6.55) | 0.485<br>(0.078-0.954) |
| 1:0.366 | 0.908<br>1.82<br>3.63<br>7.27<br>14.5 | 0.331<br>0.663<br>1.33<br>2.65<br>5.30 | 15.0%<br>36.7%<br>80.0%<br>96.7%<br>96.7% | 2.22<br>(1.88-2.62) | 0.810<br>(0.686-0.957) |
| Indomethacin Only (0:1) | 0<br>0<br>0<br>0 | 0.398<br>0.795<br>1.59<br>3.18 | 18.3%<br>30.0%<br>78.3%<br>85.0% | 0.0 | 1.08<br>(0.0012-5.14) |

# TABLE V

**ORAL NALBUPHINE/TIFLAMIZOLE COMBINATIONS**
**IN THE MOUSE ANTIPHENYLQUINONE WRITHING TEST**
**40 Min (N=28 Mice/Dose)**

| DRUG COMBINATIONS Nalbuphine HCl: Tiflamizole | DRUG DOSE (mg/kg) Nalbuphine HCl | Tiflamizole | % MICE BLOCKED | ED50 AT 40 MIN (95% Confidence Limits) Nalbuphine HCl | Tiflamizole |
|---|---|---|---|---|---|
| Control (0:0) | 0 | 0 | 3.6 | - | - |
| Nalbuphine HCl Only (1:0) | 5.45<br>10.9<br>21.8<br>43.6<br>87.2 | 0<br>0<br>0<br>0<br>0 | 10.7<br>17.9<br>32.1<br>57.1<br>82.1 | 36.2<br>(25.0-51.3) | 0.0 |
| 1:0.0246 | 4.54<br>9.08<br>18.2<br>36.3<br>72.7 | 0.112<br>0.224<br>0.448<br>0.897<br>1.79 | 17.9<br>21.4<br>67.9<br>82.1<br>100.0 | 14.9<br>(10.8-19.2) | 0.367<br>(0.266-0.423) |
| 1:0.0619 | 3.63<br>7.27<br>14.5<br>29.1<br>58.1 | 0.224<br>0.448<br>0.897<br>1.79<br>3.59 | 14.3<br>25.0<br>71.4<br>100.0<br>100.0 | 11.2<br>(8.71-13.5) | 0.692<br>(0.538-0.831) |
| 1:0.123 | 2.73<br>5.45<br>10.9<br>21.8<br>43.6 | 0.336<br>0.673<br>1.35<br>2.69<br>5.38 | 17.9<br>28.6<br>67.9<br>96.4<br>100.0 | 7.84<br>(5.78-9.85) | 0.962<br>(0.710-1.21) |
| 1:0.246 | 1.82<br>3.63<br>7.27<br>14.5<br>29.1 | 0.448<br>0.897<br>1.79<br>3.59<br>7.17 | 3.6<br>28.6<br>75.0<br>100.0<br>100.0 | 5.08<br>(4.08-6.06) | 1.25<br>(1.00-1.49) |
| 1:0.617 | 0.908<br>1.82<br>3.63<br>7.27<br>14.5 | 0.56<br>1.12<br>2.24<br>4.48<br>8.97 | 7.1<br>32.1<br>53.6<br>89.3<br>96.4 | 3.12<br>(2.34-3.96) | 1.92<br>(1.44-2.44) |
| Tiflamizole Only (0:1) | 0<br>0<br>0<br>0 | 0.673<br>1.35<br>2.69<br>5.38 | 3.6<br>32.1<br>46.4<br>82.1 | 0.0 | 2.62<br>(1.97-3.47) |

# TABLE VI

**ORAL NALBUPHINE/SULINDAC COMBINATIONS IN THE MOUSE ANTIPHENYLQUINONE WRITHING TEST**

40 MIN (N=30 Mice/Dose)

| DRUG COMBINATIONS Nalbuphine HCl: Sulindac | DRUG DOSE (mg/kg) | | % MICE BLOCKED | ED50 AT 40 MIN (95% Confidence Limits) | |
|---|---|---|---|---|---|
| | Nalbuphine HCl | Sulindac | | Nalbuphine HCl | Sulindac |
| Control (0:0) | 0 | 0 | 10.0% | - | - |
| Nalbuphine Only (1:0) | 5.45<br>10.9<br>21.8<br>43.6<br>87.2 | 0<br>0<br>0<br>0<br>0 | 16.7%<br>40.0%<br>36.7%<br>63.3%<br>90.0% | 30.8<br>(18.0-45.0) | 0.0 |
| 1:0.175 | 4.54<br>9.08<br>18.2<br>36.3<br>72.7 | 0.792<br>1.58<br>3.17<br>6.33<br>12.7 | 10.0%<br>46.7%<br>76.7%<br>96.7%<br>100.0% | 11.5<br>(8.42-14.4) | 2.01<br>(1.47-2.51) |
| 1:0.435 | 3.63<br>7.27<br>14.5<br>29.1<br>58.1 | 1.58<br>3.17<br>6.33<br>12.7<br>25.3 | 13.3%<br>30.0%<br>83.3%<br>96.7%<br>100.0% | 10.1<br>(7.52-12.4) | 4.39<br>(3.27-5.38) |
| 1:0.872 | 2.73<br>5.45<br>10.9<br>21.8<br>43.6 | 2.38<br>4.75<br>9.5<br>19.0<br>38.0 | 6.7%<br>36.7%<br>80.0%<br>96.7%<br>100.0% | 7.32<br>(5.49-8.99) | 6.39<br>(4.79-7.85) |
| 1:1.74 | 1.82<br>3.63<br>7.27<br>14.5<br>29.1 | 3.17<br>6.33<br>12.7<br>25.3<br>50.7 | 30.0%<br>20.0%<br>83.3%<br>100.0%<br>100.0% | 6.13<br>(0.00-364) | 10.7<br>(0.00-633) |
| 1:4.37 | 0.908<br>1.82<br>3.63<br>7.27<br>14.5 | 3.96<br>7.92<br>15.8<br>31.7<br>63.3 | 10.0%<br>43.3%<br>80.0%<br>100.0%<br>100.0% | 2.26<br>(1.70-2.75) | 9.87<br>(7.41-12.0) |
| Sulindac Only (0:1) | 0<br>0<br>0<br>0 | 4.75<br>9.5<br>19.0<br>38.0 | 20.0%<br>20.0%<br>46.7%<br>96.7% | 0.0 | 21.2<br>(16.6-25.2) |

# TABLE VII

ORAL NALBUPHINE/FLURBIPROFEN COMBINATIONS IN THE MOUSE ANTIPHENYLQUINONE WRITHING TEST 20 MIN (N=20-40 MICE/DOSE)

| DRUG COMBINATIONS Nalbuphine HCl: Flurbiprofen | DRUG DOSE (mg/kg) | | % MICE BLOCKED | ED50 at 20 Min. (95% Confidence Limits) | |
|---|---|---|---|---|---|
| | Nalbuphine HCl | Flurbiprofen | | Nalbuphine HCl | Flurbiprofen |
| Control (0:0) | 0 | 0 | 7.5% | - | - |
| Nalbuphine Only (1:0) | 2.93<br>5.85<br>11.7<br>23.4<br>46.8 | 0<br>0<br>0<br>0<br>0 | 12.5%<br>22.5%<br>45.0%<br>67.5%<br>80.0% | 16.5 (11.6-22.4) | 0.0 |
| 1:0.027 | 2.44<br>4.88<br>9.75<br>19.5<br>39.0 | 0.067<br>0.133<br>0.267<br>0.533<br>1.07 | 27.5%<br>20.0%<br>62.5%<br>97.5%<br>97.5% | 9.07 (0.01-974) | 0.245 (0.000-26.3) |
| 1:0.0683 | 1.95<br>3.9<br>7.8<br>15.6<br>31.2 | 0.133<br>0.267<br>0.533<br>1.07<br>2.13 | 12.5%<br>45.0%<br>70.0%<br>92.5%<br>100.0% | 5.21 (4.00-6.43) | 0.356 (0.273-0.439) |
| 1:0.137 | 1.46<br>2.93<br>5.85<br>11.7<br>23.4 | 0.2<br>0.4<br>0.8<br>1.6<br>3.2 | 12.5%<br>30.0%<br>75.0%<br>97.5%<br>100.0% | 4.24 (3.35-5.05) | 0.581 (0.459-0.693) |
| 1:0.274 | 0.975<br>1.95<br>3.9<br>7.8<br>15.6 | 0.267<br>0.533<br>1.07<br>2.13<br>4.27 | 15.0%<br>40.0%<br>80.0%<br>100.0%<br>97.5% | 2.39 (0.293-4.59) | 0.654 (0.080-1.26) |
| 1:0.683 | 0.488<br>0.975<br>1.95<br>3.9<br>7.8 | 0.333<br>0.667<br>1.33<br>2.67<br>5.33 | 25.0%<br>25.0%<br>65.0%<br>90.0%<br>95.0% | 1.58 (1.14-2.03) | 1.08 (0.781-1.39) |
| Flurbiprofen Only (0:1) | 0<br>0<br>0<br>0 | 0.4<br>0.8<br>1.6<br>3.2 | 5.0%<br>32.5%<br>45.0%<br>87.5% | 0.0 | 1.56 (1.17-1.97) |

WHAT IS CLAIMED IS: BP-6213 Cognate

1. A pharmaceutical composition comprising a synergistically effective combination of analgesically effective amounts of nalbuphine, or a pharmaceutically suitable salt thereof, and a non-narcotic analgesic compound selected from the group consisting of ibuprofen, zomepirac, indomethacin, tiflamizole, sulindac, and flurbiprofen, or a pharmaceutically suitable salt thereof.

2. The composition of Claim 1 wherein the nalbuphine is present as the hydrochloride salt.

3. The composition of Claim 1 wherein said non-narcotic analgesic compound is present as the sodium salt.

4. The composition of Claim 1 wherein said non-narcotic analgesic compound is ibuprofen and the weight ratio of nalbuphine:ibuprofen is from about 1:0.40 to about 1:79.

5. The composition of Claim 1 wherein said non-narcotic analgesic compound is zomepirac and the weight ratio of nalbuphine:zomepirac is from about 1:0.02 to about 1:26.

6. The composition of Claim 1 wherein said non-narcotic analgesic compound is indomethacin and the weight ratio of nalbuphine:indomethacin is from about 1:0.01 to about 1:20.

7. The composition of Claim 1 wherein said non-narcotic analgesic compound is tiflamizole and the weight ratio of nalbuphine:tiflamizole is from about 1:0.0046 to about 1:0.62.

8. The composition of Claim 1 wherein said non-narcotic analgesic compound is sulindac and the weight ratio of nalbuphine:sulindac is from about 1:0.1 to about 1:400.

9. The composition of Claim 1 wherein said non-narcotic analgesic compound is flurbiprofen and the weight ratio of nalbuphine:flurbiprofen is from about 1:0.027 to about 1:15.

10. The composition of Claims 1 to 10 further comprising the addition of a suitable pharmaceutical carrier.

11, A method of preparation of a pharmaceutical composition comprising combining an analgesically effective amount of nalbuphine, or a pharmaceutically suitable salt thereof, and a synergistically effective amount of a non-narcotic analgesic compound selected from the group consisting of ibuprofen, zomepirac, indomethacin, tiflamizole, sulindac, and flurbiprofen, or a pharmaceutically suitable salt thereof, together with the suitable vehicles, excipients, carriers, and co-helpers for each form of composition.

FIGURE 1

ISOBOLOGRAM FOR THE INTERACTION OF ORAL NALBUPHINE HCl AND IBUPROFEN IN THE MOUSE ANTIPHENYLQUINONE WRITHING TEST

(5 MIN.)

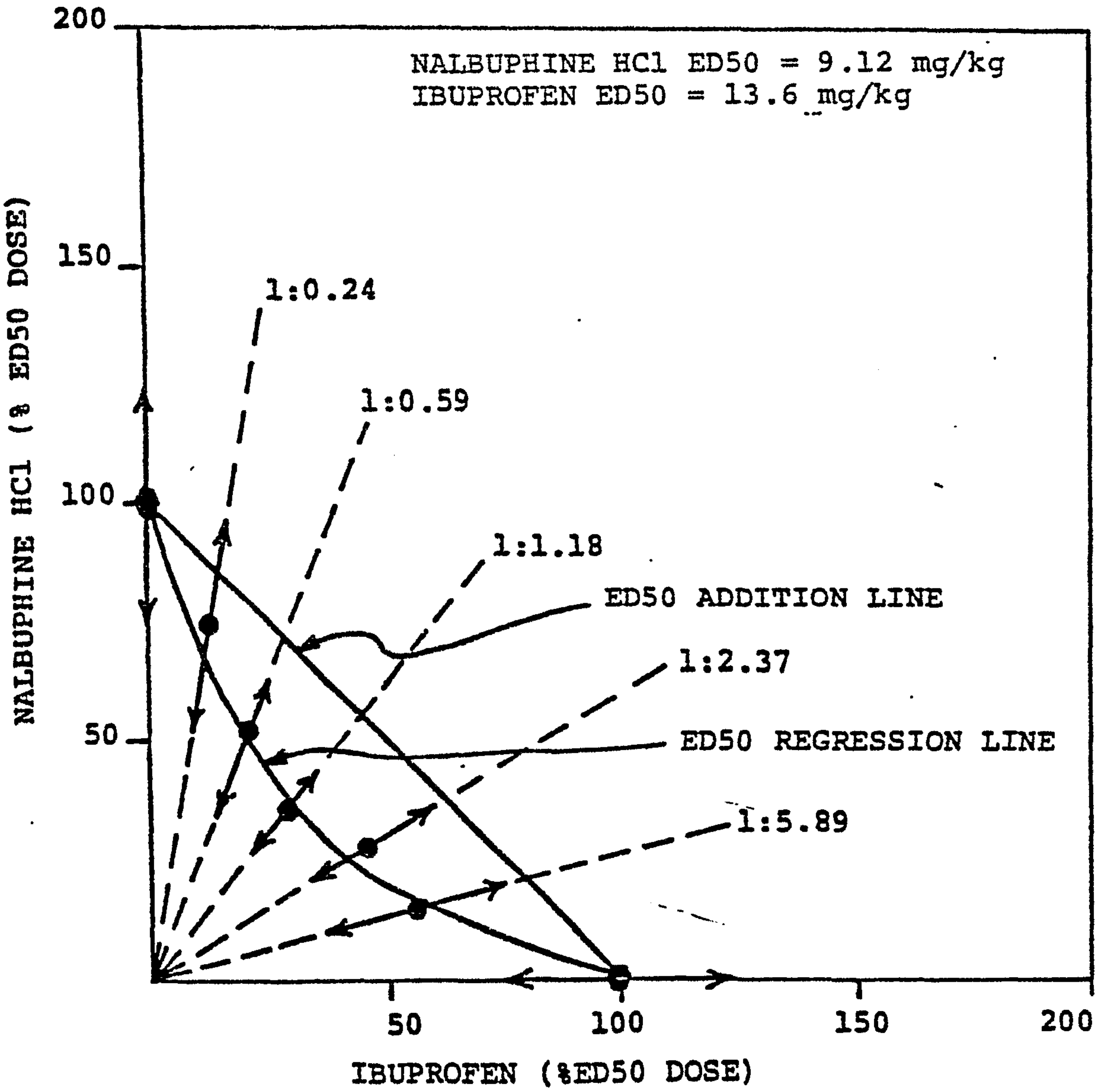

FIGURE 2

ISOBOLOGRAM FOR THE INTERACTION OF ORAL NALBUPHINE HCl AND ZOMEPIRAC SODIUM IN THE MOUSE ANTIPHENYLQUINONE WRITHING TEST

(20 MIN)

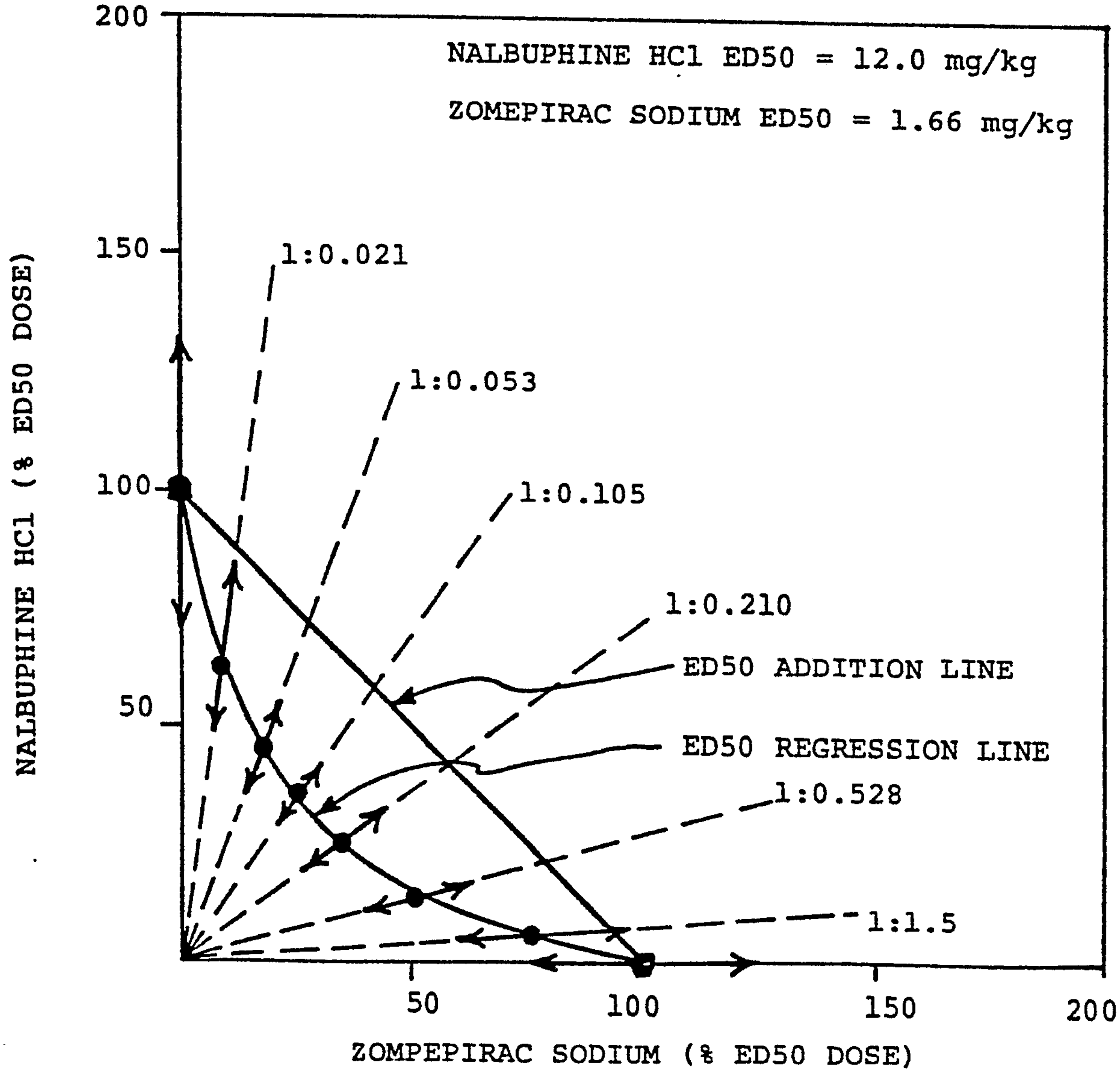

FIGURE 3

ISOBOLOGRAM FOR THE INTERACTION OF ORAL NALBUPHINE HCl AND INDOMETHACIN IN THE MOUSE ANTIPHENYLQUINONE WRITHING TEST

(40 MIN)

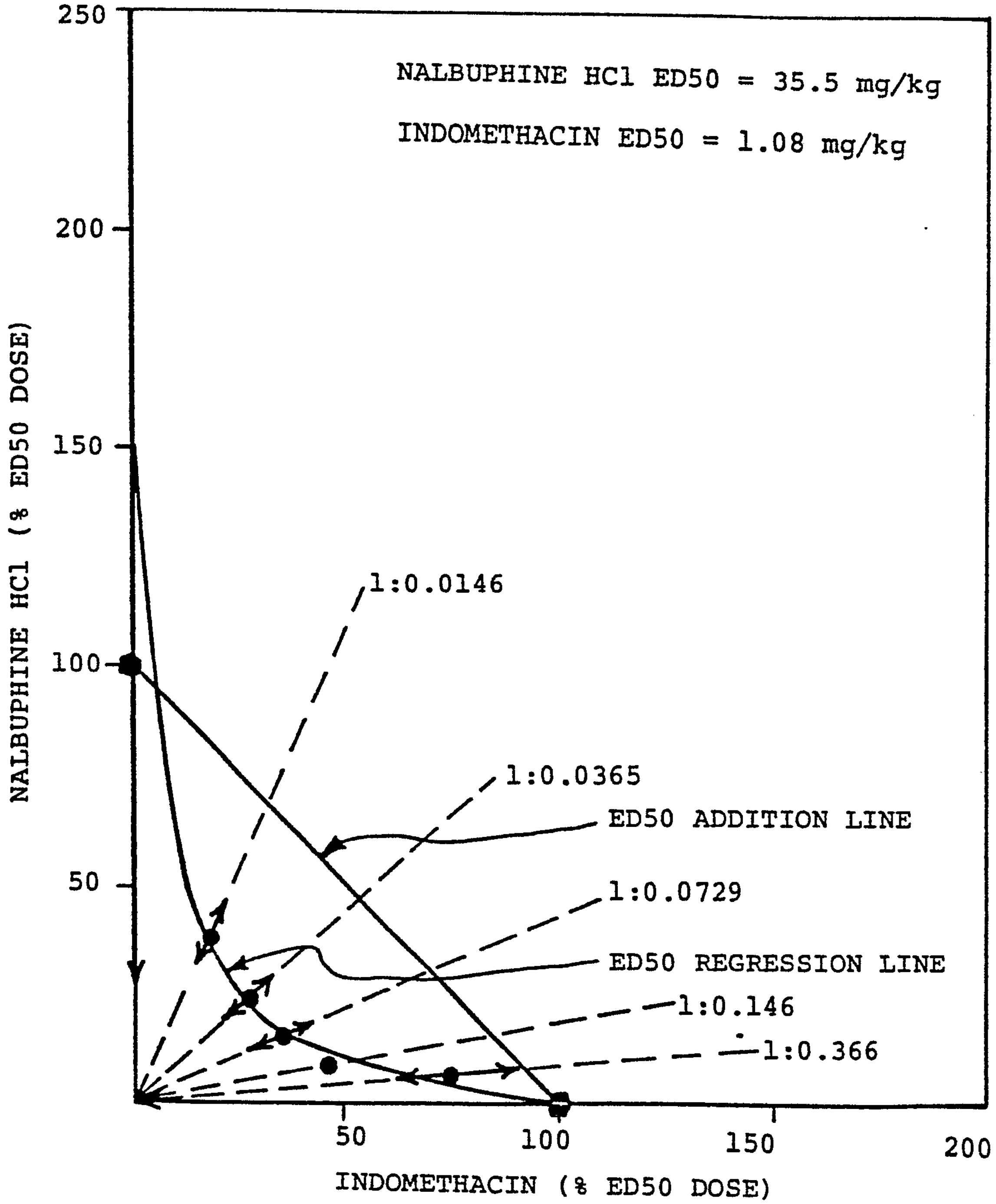

FIGURE 4

ISOBOLOGRAM FOR THE INTERACTION OF

ORAL NALBUPHINE HCl AND TIFLAMIZOLE

IN THE MOUSE ANTIPHENYLQUINONE WRITHING TEST

(40 MIN)

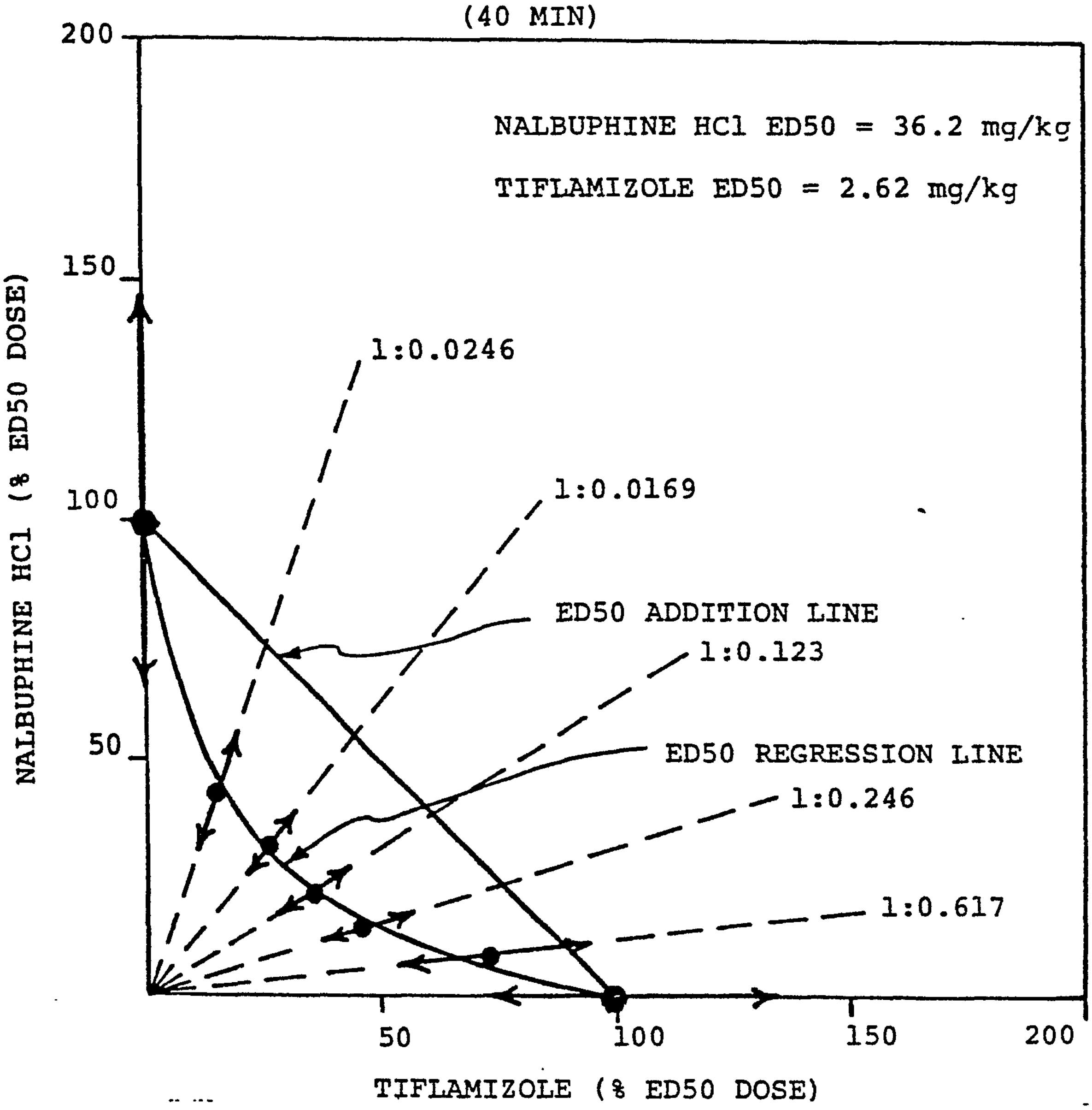

FIGURE 5

ISOBOLOGRAM FOR THE INTERACTION OF
ORAL NALBUPHINE HCl AND SULINDAC IN
THE MOUSE ANTIPHENYLQUINONE WRITHING TEST
(40 MIN)

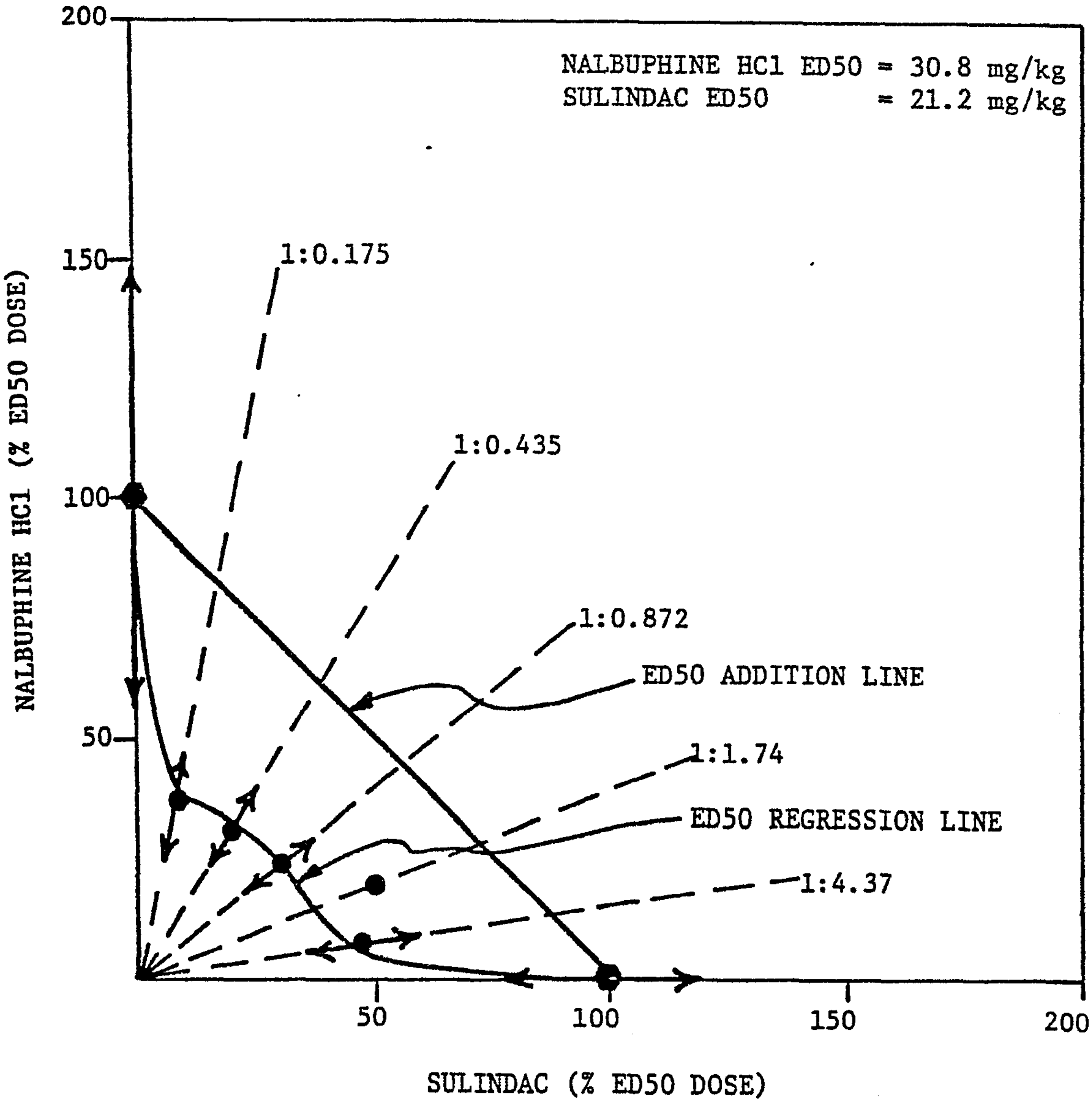

FIGURE 6

ISOBOLOGRAM FOR THE INTERACTION OF
ORAL NALBUPHINE HCl AND FLURBIPROFEN IN
THE MOUSE ANTIPHENYLQUINONE WRITHING TEST
(20 min)